# EUROPEAN PATENT APPLICATION

(11) **EP 2 975 018 A1**
(43) Date of publication of application: **20.01.2016**
(21) Application number: 14177418.2
(22) Date of filing: 17.07.2014
(51) Int. Cl.: C07C 201/00, C07C 207/04, C07C 231/02

(54) **Method of nitrosation of phenol**

(71) Applicant: DSM Fine Chemicals Austria Nfg GmbH & Co KG, 4021 Linz (AT)
(72) Inventor: Hermsen, Petrus, Johannes, 6100 AA Echt (NL); Poechlauer, Peter, 6100 AA Echt (NL)
(74) Representative: Hansen, Jesper Römer

(57) **Abstract**

Method of nitrosation of phenol, involving continuous preparation of dinitrogen trioxide in a microreactor, and reaction of dinitrogen trioxide with the aromatic organic compound.

## Description

The invention relates to a method of nitrosation of a phenol. More particularly, the invention relates to nitrosation of a phenol in the manufacturing of 4-nitrosophenol and optionally reacting the thus formed 4-nitrosophenol further to form N-(4-hydroxyphenyl)acetamide (APAP).

N-(4-hydroxyphenyl)acetamide also referred to as paracetamol is an active pharmaceutical ingredient for example in pain killers. Various routes for the synthesis of paracetamol have been identified including the route involving nitrosation of phenol.

RO2108864 discloses a process for the preparation of p-nitrosophenol from phenol, sodium nitrite and sulfuric acid. This process produces salts in addition to the desired nitrosophenol, which in the process according to the invention is considered a waste products.

RU 2129117 concerns a method of preparation of p-nitrosophenol from nitrogen trioxide and phenol. This patent discloses and claims the nitrosation of a suspension of phenol in water by N₂O₃ in the presence of a catalyst. RU 2127117 does not disclose continuous preparation of N₂O₃ and discloses that presence of ammonium chloride or dilute hydrochloric acid is required as catalyst.

RU2461543 discloses a method of manufacturing paracetamol via a process involving reduction of p-nitrosophenol in ethyl acetate in the presence of a Pd/C-containing catalyst by hydrogen at 2.0-4.0 atm and temperature 20-50°, followed by acylation of the intermediate p-aminophenol and the end product recovery.

FR2533559B1 discloses a process for the preparation of N-acetyl-p-aminophenol by simultaneous hydrogenation and acetylation of nitrosophenol in a solvent medium comprising at least one ester of an alcohol containing from 1 to 6 carbon atoms and/or at least one aliphatic organic acid containing from 1 to 5 carbon atoms.

It is an object of the invention to provide an improved method of manufacturing 4-NO phenol.

It is further object of the invention to provide an improved method of manufacturing N-(4-hydroxyphenyl)acetamide (APAP).

The improvement may for example be one or more of improved process mass intensity, improved safety, and/or reduced environmental impact.

One or more of the above and/or other objects of the invention are realized by a method according to claim 1.

In another aspect of the invention, one or more of the above and/or other objects of the invention are realized by a method according to claim 7.

### Brief description of the drawings

The invention will be explained more fully below with reference to exemplary embodiments as well as the figures, in which
Figure 1 shows the experimental set-up allowing the continuous generation of N₂O₃ and subsequent nitrosation of phenol, and
Figure 2 shows the experimental set-up allowing the *in situ* generation of N₂O₃ and subsequent nitrosation of phenol.

The terms N-acetyl-p-aminophenol, N-(4-hydroxyphenyl)acetamide, paracetamol and APAP refers to the same chemical compound and are used interchangeably herein.

Process mass intensity (PMI) is herein meant as the total mass of materials used to produce a specified mass of product. Materials include reactants, reagents, catalysts and solvents used for reaction, isolation and purification (Org.Proc.Res.Dev. 2011, 15, 912).

The process according to the invention involves nitrosation of phenol to form 4-nitrosophenol. The nitrosation is conducted by reacting phenol with dinitrogen trioxide, N₂O₃, which N₂O₃ has been prepared in a continuous reaction between nitrogen monoxide, NO, and oxygen, O₂. Preferably, NO and O₂ are fed to a tube flow reactor and reacted at reduced temperature, such as -50°C to 0°C. Gaseous NO and O₂ are fed continuously to the reactor while N₂O₃ is removed in liquid form due to the low temperature and optionally collected before further use. The use of tube flow reactor allows for a very low reactor volume and hence provides for a safe working environment where only small amounts of the toxic and air pollutant NO is present in the reactor at any time. The reaction between NO and O₂ may be conducted at ambient pressure, but it is preferred to conduct the reaction at between 2 to 10 bar to speed up the reaction. During the reaction between NO and O₂, additional (inert) gasses may be present, such as N₂.

By reacting nitrogen monoxide with oxygen in a continuous process is herein meant that the reaction in which new materials (NO and O₂) are added and product (N₂O₃) removed continuously at a rate that maintains the reaction volume at a specific level. In other words, continuous reactors that may be used carry out steady state operations.

A microreactor is generally defined as a miniaturized reactor with characteristic dimensions (channel of plate width) in micrometers to millimeters (preferably from 0.01 mm to 10.0 mm). A number of microreactors may be combined in parallel to form a micro-structured reactor. Thus, the volume available for reaction depends on the diameter and length of the microreactor, or in case of a micro-structured reactor is used on the dimensions of the parallel channels and the number of parallel channels. The volume of microreactors or micro-structured reactors typically lies in the range of 1 ml to 1 m³, where 1m³ correspond to a very large number of parallel channels in a micro-structured reactor. Preferably, the volume of the microreactors or micro-structured reactors are from 10 ml to 50 l.

Preferably, the microreactor has a channel with a hydraulic diameter of 20 mm or less. The hydraulic diameter Dh is defined as 4 A/U, wherein A is the inner cross sectional area of the reactor channel and U is the perimeter of said cross section. For a round tube, the hydraulic diameter equals the inner diameter of the tube. For a rectangular duct, that has a cross section with a rectangular shape, the hydraulic diameter equals 4 LW/2(L+W), wherein L is the length of the longest side of the rectangle and W is the width of the reactangle. For the special case of a square duct the hydraulic diameter equals L. It should be noted that the general formula 4 A/U allows the calculation of the hydraulic diameter for any shape of reactor channel.

In the present invention, it is preferred that the microreactor is a tubular flow reactor because these are readily available at a wide range of materials and dimensions, Furthermore, tubular flow reactors are easily scalable for example via modular building, which facilitate replacement of one or more modules optionally inline, thereby allowing maximum flexibility.

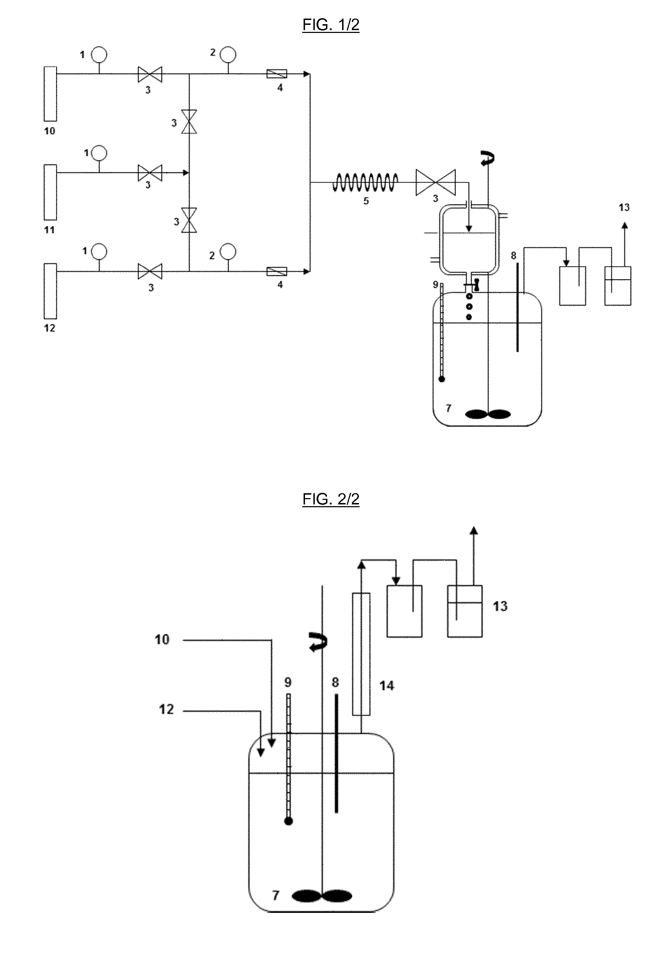

In Figure 1, an example of an experimental setup suitable for conducting the process according the invention where N₂O₃ is generated continuous and thereafter used for nitrosation of the phenol is shown. The raw materials, oxygen gas, 10, and nitric oxide, 12, are supplied via individual pressure indicator and controllers, 1, and two-way valves, 3. The mass flow to the tubular flow reactor, 5, is determined by indicator and controller, 2, and enters the tubular flow reactor, 5, via back pressure valve 4, which prevent back flow of reacted reactants.

In the tubular flow reactor, 5, oxygen and nitric oxide reacts to form N₂O₃ in liquid state, which N₂O₃ is continuously flowed via another two-way valve, 3, to a cooled double-jacketed dropping funnel, 6. From the dropping funnel, 6, the N₂O₃ is added controlled to a stirred batch reactor, 7, containing phenol solution, in which reactor N₂O₃ reacts with the phenol to form nitrosophenol. The conditions of the batch reactor, 7, is monitored by thermometer, 9, and pH-electrode, 8.

Unreacted gas, 13, is exhausted from the batch reactor via scrubbers containing aqueous base such as NaOH. After completion of the nitrosation of the phenol, the reaction product (nitrosophenol) is isolated from the reaction mixture by filtration and optionally purified by recrystallization. Furthermore, the tubular flow reactor may be purged for example by nitrogen gas, 11, via dedicated two-way valve, 3, pressure indicator and controller, 1.

In Figure 2, an example of an experimental setup suitable for conducting the process according the invention with in-situ generation of N₂O₃ and subsequent nitrosation of phenol is shown. Nitric oxide, 12, and oxygen, 10, is supplied via individual pressure indicators, controllers, two-way valves, and back pressure valves (not shown) to a stirred batch reactor, 7, containing phenol solution. The N₂O₃ is generated in the batch reactor and thereafter reacts (also in the batch reactor, 7) with phenol previously supplied to the batch reactor, 7. The batch reactor, 7, is provided with a reflux condenser, 14, which recycles condensable elements of the exhaust gas before the exhaust gas enters the scrubber, 13.

After completion of the nitrosation of the phenol, the reaction product (nitrosophenol) is extracted from the reaction mixture by filtration and optionally purified by recrystallization.

The reaction between N₂O₃ and phenol may be conducted in various hydrophilic solvents like methanol, ethanol and water, but it was found to be advantages to use an aqueous solution as it was surprisingly found that for aqueous solutions it was possible to conduct the reaction without the presence of a catalyst, which allows for an environmentally and economical advantage as compared to previous processes for nitrosation of phenol. By catalyst is herein understood a compound added to enhance reaction speed without being consumed by the reaction.

After the reaction between N₂O₃ and phenol, the resulting 4-nitrosophenol is preferably separated from the reaction mixture by filtration. It is preferred to conduct this separation at reduced temperature, such as at a temperature below 10°C and preferably at a temperature between -10 to 5°C. It was found that a yield of 4-nitrosophenol preferably should be at least 65 mol% based on the amount of phenol. More preferably the yield of 4-nitrosophenol should be at least 75 mol%. Since the reaction between N₂O₃ and phenol will lead to some side reactions, it is preferred that the yield of the filtration is kept below 95 mol% and more preferably the yield is kept below 90 mol%.

Surprisingly it was found that using the method of the invention to manufacture 4-nitrosophenol without catalyst allowed for low process mass intensity. Particularly it was found to be advantageous to conduct the method of manufacturing 4-nitrosophenol starting from phenol, nitrogen monoxide, oxygen and using water as solvent with a process mass intensity of less than 25, more preferably with a process mass intensity of less than 15, even more preferably with a process mass intensity of less than 10. In other words, the process according to the invention therefore represents a major advantage due to the very low environmental impact of the process as compared to methods of manufacturing 4-nitrosophenol till now.

Another aspect of the invention concerns a method of manufacturing aminophenol by hydrogenating 4-nitrosophenol obtained according to a method of the first aspect of the invention. It was found to be advantageous that the step of hydrogenating is conducted by reacting 4-nitrosophenol dissolved in a C1-C5 alcohol with H₂ gas in the presence of a catalyst. Preferably the C1-C5 alcohol contains at least 50 wt% methanol, such as 75 wt% or 90 wt%, and more preferably the C1-C5 alcohol is methanol. By C1-C5 alcohol is herein meant an alcohol with 1 to 5 carbon atoms, including methanol, ethanol, propanol, butanol, pentanol, Ethane-1,2-diol, Propane-1,2-diol, Propane-1,2,3-triol, Butane-1,2,3,4-tetraol, Pentane-1,2,3,4,5-pentol, isomers thereof and mixtures thereof.

The catalyst used in the hydrogenation may be any commonly used hydrogenation catalyst, however it is preferred to use a Pd/C catalyst, as this catalyst was found to be very active for this reaction.

A further aspect of the invention concerns method of manufacturing APAP by acylating aminophenol obtained by a method of the second aspect of the invention. Preferably the acylation is carried out by reacting aminophenol dissolved in a C1-C5 alcohol with acetic anhydride, Ac₂O. The C1-C5 alcohol preferably contains at least 50 wt% methanol, such as 75 wt% or 90 wt%, and more preferably the C1-C5 alcohol is methanol. Alternative acylation agents that can be used, such as for example (but not limited to) acetyl chloride, acetyl bromide, methyl acetate, vinyl acetate and acetic acid, however, these may require presence of a catalyst during the process.

In a highly preferred embodiment, the hydrogenating of 4-nitrosophenol and acylating of aminophenol are conducted in a one pot reaction. By a one pot reaction is herein meant that the reaction is conducted in one reactor and at the same time.

Surprisingly it was found that using the method according to this aspect of the invention allowed for very low process mass intensity. Particularly it was found to be advantageous to conduct the method of manufacturing APAP starting from phenol, nitrogen monoxide, oxygen and acetic anhydride and using water and methanol as solvents (which solvents were recycled) with a process mass intensity of less than 40, more preferably with a process mass intensity of less than 30, even more preferably with a process mass intensity of less than 20. In other words, the process according to the invention therefore represents a major advantage due to the very low environmental impact of the process as compared to methods of manufacturing APAP till now.

### EXAMPLES

### Example 1: Synthesis of dinitrogen trioxide

Using the equipment depicted in Fig. 1 liquid dinitrogen trioxide was generated by mixing gaseous nitrous oxide (240 Nml/min) and oxygen (30 Nml/min) in a cooled (-20°C) tubular flow reactor (Teflon, volume 12 mL, internal diameter 2.4 mm) at atmospheric pressure. The dinitrogen trioxide was collected in a cooled (-35°C), double jacketed dropping funnel from which the ink-blue liquid can be added to a stirred reactor containing an aqueous solution of phenol.
Examples 1 shows that it is possible to synthesize liquid dinitrogen trioxide by use of a tubular flow reactor continuously.

### Example 2: Synthesis of 4-nitrosophenol

A 2.0 L solution of phenol in water (94.11 g phenol, 0.5 M) was cooled to 0°C and blue liquid N₂O₃ (generated according to Example 1; 600 mmol, 0.6 equiv) was added over a period of 5 hours. The precipitated brow product was isolated through filtration over a glass filter and washed with 200 mL of cold water (0°C). After drying (50°C) 85.66 grams (70mol% yield) of 4-nitrosophenol was obtained. The amount of 2-nitrosophenol present in this material was below the detection limit.

Examples 2 shows that it is possible to synthesize 4-nitrosophenol without the need for any additional catalyst..

### Example 3: Synthesis of N-acetyl 4-amino phenol (APAP)

4-nitrosophenol (87.8 g) was dissolved in 878 mL methanol (11 wt% solution). To this solution, Ac₂O (2.0 equiv) and 4.39 grams Pd/C (5 wt%) were added. This mixture was brought under H₂ atmosphere (20 bar) and stirred for 1 hour at 50 °C showing complete conversion toward desired APAP. The crude reaction mixture obtained, consisted of >98.5% APAP and trace amounts of 4-aminophenol and over-acylated APAP.

The catalyst was removed by filtration and subsequently 100 mL of water was added to the filtrate. Methanol was removed *in vacuum* and the resulting mixture was cooled on ice. Crystallization of APAP was initiated by seeding with authentic APAP. The desired product was isolated as dark brown crystals upon filtration and washing with cold water. After drying 88 g (81.6mol%) of APAP was obtained.

Examples 3 shows that it is possible to synthesize N-acetyl 4-amino phenol with a high yield using methanol as solvent.

### Example 4: Synthesis of 4-nitrosophenol

The nitrosation of phenol using *in situ* formed dinitrogen trioxide was investigated in the experimental set-up depicted in Fig. 2.

NO gas was used as a gas cap (generated by constant feeding to an aqueous solution of phenol), and O₂ gas was injected in this gas layer at lower addition rate. Several different conditions were tried by changing the gas ratios and by using pH control (pH-stat). The results of these experiments are depicted in the table below (Table 1). In all cases 240 mL of a 0.5 M solution of phenol in water was used at ambient temperature.

**Table 1. GC analyses of the crude reaction mixtures at the stated reaction times.**

| Exp. | NO NmL/min | O2 NmL/min | Time (min) | pH | Peak Area % | | | | | |
|---|---|---|---|---|---|---|---|---|---|---|
| | | | | | Phenol | 4-NO-phenol | 4-NO2-phenol | 2-NO2-phenol | 2,4-(NO2)2-phenol | 1.4-benzo-quinone |
| 1 | 50 | 5 | 40 | 1.45 | 49.5 | 0.0 | 48.9 | 1.6 | 0.0 | 0.0 |
| | 50 | 5 | 100 | 0.89 | 2.7 | 0.0 | 48.3 | 2.9 | 25.5 | 20.6 |
| 2 | 100 | 5 | 70 | 1.61 | 68.7 | 25.0 | 3.6 | 2.1 | 0.0 | 0.7 |
| | 100 | 5 | 100 | 1.04 | 41.0 | 14.8 | 5.2 | 9.5 | 0.0 | 3.9 |
| 3 | 100 | 0 | 66 | 2.35 | 97.6 | 2.4 | 0.0 | 0.0 | 0.0 | 0.0 |
| 4 | 100 | 5 | 200 | 2.5 (pH stat) | 75.7 | 23.9 | 0.0 | 0.4 | 0.0 | 0.0 |
| | 100 | 5 | 300 | 2.5 (pH stat) | 63.5 | 35.2 | 0.0 | 1.0 | 0.0 | 0.2 |
| 5 | 100 | 5 | 85 | 3.5 (pH stat) | 89.9 | 8.1 | 0.0 | 0.8 | 0.0 | 0.0 |
| | 100 | 5 | 450 | 3.0 (pH stat) | 25.9 | 34.1 | 13.6 | 12.1 | 0.0 | 3.5 |

| | | | | | | | | | | |
|---|---|---|---|---|---|---|---|---|---|---|
| Examples 4 shows that in situ formation of dintrogen trioxide allows the synthesis of p-nitrosophenol. | | | | | | | | | | |

### Example 5: Synthesis of N-acetyl 4-amino phenol (APAP)

The hydrogenation and acylation of crude 4-nitrosophenol was investigated using an Argonaut Endeavor autoclave.

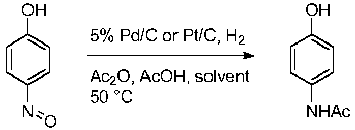

In this screening, four different solvents and two different catalysts were tested under conditions as indicated in Table 2.

**Table 2. Endeavor hydrogenation/acylation screenings experiments**

| Entry^{a} | Temp. | H₂ | Catalyst^{b} | Solvent | Solvent | AcOH | Ac₂O | APAP | *O*-Ac-APAP |
|---|---|---|---|---|---|---|---|---|---|
| | °C | bar | 10 wt% | | mL | mL | 1.5 equiv | % | % |
| 1 | 50 | 20 | Pd/C | AcOH | 6 | 0 | 230 µL | 99.5 | 0.5 |
| 2 | 50 | 20 | Pd/C | MeOH | 5 | 1 | 230 µL | 100 | 0 |
| 3 | 50 | 20 | Pd/C | THF | 5 | 1 | 230 µL | 96.5 | 3.5 |
| 4 | 50 | 20 | Pd/C | IPAT | 5 | 1 | 230 µL | 98.1 | 1.9 |
| 5 | 50 | 20 | Pt/C | AcOH | 6 | 0 | 230 µL | 99.5 | 0.5 |
| 6 | 50 | 20 | Pt/C | MeOH | 5 | 1 | 230 µL | 100 | 0 |
| 7 | 50 | 20 | Pt/C | THF | 5 | 1 | 230 µL | 97.8 | 2.2 |
| 8 | 50 | 20 | Pt/C | IPAT | 5 | 1 | 230 µL | 98 | 2 |

| | | | | | | | | | |
|---|---|---|---|---|---|---|---|---|---|
| ^{a} Hydrogenation conditions: All reactions were conducted using 200 mg substrate (4-nitroso phenol; obtained in experiment 2), 10 wt% catalyst (20 mg) in 5 mL of solvent, 1 mL AcOH and 230 µL Ac₂O (1.5 equiv) at 50°C and 20 bar hydrogen pressure, 1 hour. ^{b} Pd/C: 5% on activated wood carbon, reduced, 50% H₂O wet paste (Escat™ 1421). | | | | | | | | | |

Examples 5 shows that hydrogenation of 4-nitrosophenol in the presence of acetic anhydride yields APAP in a single step. The best result was obtained in an alcoholic solvent.

## Claims

1. A method of manufacturing 4-nitrosophenol, comprising the steps of
- reacting nitrogen monoxide, NO, with oxygen, O₂, to form dinitrogen trioxide, N₂O₃,
- optionally collecting the formed dinitrogen trioxide, N₂O₃,
- reacting dinitrogen trioxide with phenol in water to obtain a 4-nitrosophenol, wherein reacting nitrogen monoxide with oxygen is a continuous process.

2. Method according to claim 1, wherein reacting nitrogen monoxide with oxygen in the continuous process is conducted in a tubular flow reactor at a temperature of -50°C to 0°C to form liquid dinitrogen trioxide.

3. Method according to any one of the claims 1 or 2, wherein the step of reacting dinitrogen trioxide with phenol is conducted in an aqueous solution without present of a catalyst.

4. Method according to any one of claims 1 to 3, further comprising the step of separating 4-nitrosophenol by filtration, preferably the yield of 4-nitrosophenol is at least 65 mol% based on the sum of initial N₂O₃ and phenol.

5. Method according to any one of the claims 1 to 4, starting from phenol, nitrogen monoxide, oxygen and acetic anhydride using water and/or methanol as solvents with a process mass intensity with a process mass intensity of less than 25, more preferably with a process mass intensity of less than 15.

6. Method of manufacturing aminophenol comprising the step of hydrogenating 4-nitrosophenol obtained according to any one of the claims 1 to 5, preferably the step of hydrogenating is by reacting 4-nitrosophenol dissolved in a C1-C5 alcohol with H₂ gas in the presence of a catalyst, more preferably the C1-C5 alcohol contains at least 50 wt% methanol and more preferably the C1-C5 alcohol is methanol.

7. Method according to claim 6, wherein the catalyst is a Pd/C catalyst.

8. Method of manufacturing APAP comprising the step of acylating aminophenol obtained according to any one of claims 6 or 7, preferably by reacting aminophenol dissolved in a C1-C5 alcohol with acetic anhydride, Ac₂O, preferably the C1-C5 alcohol contains at least 50 wt% methanol and more preferably the C1-C5 alcohol is methanol.

9. Method according to claim 8, wherein hydrogenating 4-nitrosophenol and acylating aminophenol are conducted in a one pot reaction.

10. Method of manufacturing APAP according to any one of claims 8 or 9, starting from phenol, nitrogen monoxide, oxygen and acetic anhydride and using water and methanol as solvents under recycling of methanol, and water with a process mass intensity of less than 40, more preferably with a process mass intensity of less than 30, even more preferably with a process mass intensity of less than 20.
